(19) Europäisches Patentamt
European Patent Office
Office européen des brevets

(11) Numéro de publication : **0 030 496**
**B1**

(12) **FASCICULE DE BREVET EUROPÉEN**

(45) Date de publication du fascicule du brevet : **13.07.83**

(51) Int. Cl.³ : **C 07 G 7/00**, A 61 K 39/08, A 61 K 39/385

(21) Numéro de dépôt : **80401708.5**

(22) Date de dépôt : **28.11.80**

(54) **Composé polypeptidique thiolé provenant d'un fragment de la toxine tétanique, son procédé d'obtention et ses applications.**

(30) Priorité : **28.11.79 FR 7929289**

(43) Date de publication de la demande :
**17.06.81 Bulletin 81/24**

(45) Mention de la délivrance du brevet :
**13.07.83 Bulletin 83/28**

(84) Etats contractants désignés :
**BE CH DE GB IT LI NL**

(56) Documents cités :
**DE A 1 617 880**
**FR A 2 270 891**
**FR A 2 334 954**
**FR A 2 366 569**
**GB A 1 492 445**
**GB A 2 013 690**
**US A 3 171 831**

**JOURNAL OF NEUROCHEMISTRY, vol. 28 (3), 1977, pages 529-542 Oxford, GB B. BIZZINI et al. : « An antigenic polypeptidefragment isolated from tetanus Toxin... »**

**BIOCHEMISTRY, vol. 17, no. 8, 1978 Easton P. A. US TE PIAO KING et al. : « Preparation of protein conjugates via intermolecular disulfide bond formation » pages 1499-1506**

**Le dossier contient des informations techniques présentées postérieurement au dépôt de la demande et ne figurant pas dans le présent fascicule.**

(73) Titulaire : **INSTITUT PASTEUR**
**25-28, rue du Docteur Roux**
**F-75724 Paris Cedex 15 (FR)**

(72) Inventeur : **Bizzini, Bernard**
**12, rue Sarrette**
**F-75014 Paris (FR)**

(74) Mandataire : **Phélip, Bruno et al**
**c/o Cabinet Harlé & Phélip 21, rue de La Rochefoucauld**
**F-75009 Paris (FR)**

**EP 0 030 496 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

Composé polypeptidique thiolé provenant d'un fragment de la toxine tétanique, son procédé d'obtention et ses applications

La présente invention concerne un composé polypeptidique thiolé provenant d'un fragment de la toxine tétanique, son procédé d'obtention et ses applications, notamment à titre d'agent de transport neuropharmacologique et de marquage spécifique des cellules neuronales.

En ce qui concerne la toxine tétanique elle-même ou son anatoxine on a déjà proposé de les utiliser notamment pour l'obtention de vaccins et de réactifs de dosage. Pour illustrer cet état de la technique on citera les documents ci-après :

— le certificat d'addition FR 74.16.936 publié sous le n° 2.270.891, qui concerne un procédé pour l'obtention de vaccins par traitement d'un produit toxique avec du glutaraldéhyde. Ce procédé consiste à effectuer le traitement au glutaraldéhyde en réalisant à la fois une polymérisation d'un nombre limité de molécules du produit toxique et une détoxification dudit produit. Dans le procédé le produit toxique mis en œuvre peut être la toxine tétanique ;

— la demande de brevet FR 77.29.186 publiée sous le n° 2.366.569 concerne un procédé immunochimique de dosage d'haptènes, qui consiste à utiliser une particule sensibilisée par un anticorps, préparée en sensibilisant de fines particules par l'anticorps de l'haptène à doser et un conjugué haptène-support. Le support de ce conjugué peut être notamment le toxoïde du tétanos. Le conjugué haptène-support est utilisé comme réactif dans le procédé immunochimique et également pour l'immunisation d'un animal en vue de l'obtention de l'anticorps correspondant (voir page 4, lignes 20 à 32). Le toxoïde du tétanos est donc utilisé comme support de l'haptène dans l'organisme de l'animal de l'animal en vue de l'obtention de l'anticorps. Cependant, rien n'indique dans cette demande de brevet qu'une fraction particulière de la toxine tétanique peut être utilisée comme agent de transport axonal de médicament ;

— le brevet GB 2.013.690 concerne un antigène pour le test précoce de grossesse. Cet antigène est obtenu à partir de la sous-unité bêta de la gonadotrophine chorionique humaine, par réduction et clivage de 3, 4, 5 ou 6 ponts disulfures intrachaîne de ladite sous-unité bêta, alkylation des groupes disulfures ainsi réduits. Cet antigène peut être ensuite couplé avec une protéine ou un haptène pour augmenter sa spécificité immunologique. Le toxoïde du tétanos est cité à titre de protéine appropriée ;

— le brevet GB 1.492.445 concerne une composition contenant un conjugué constitué d'un support immunogéniquement compatible et d'un dérivé de la sous-unité bêta de la gonadotrophine chorionique humaine. Le toxoïde du tétanos est utilisé comme support ;

— la demande de brevet DE-OS 1.617.880 concerne un procédé pour obtenir des substances bioactives organotropes, en particulier des médicaments. Ce procédé consiste à faire un conjugué d'une substance biologiquement active avec des substances organotropes réceptives obtenues à partir de membranes cellulaires ou d'anticorps.

A titre de substances organotropes, sont citées notamment les toxines.

Par ailleurs, on a proposé d'utiliser des protéines thiolées à titre de véhicules pour les médicaments. A cet effet on peut citer le brevet US 3.171.831 qui concerne la thiolation de protéines par réaction avec la thiolactone d'homocystéine en présence d'une amine tertiaire. Les protéines thiolées ainsi obtenues, par exemple la gélatine, peuvent être utilisées à titre de véhicules pour les médicaments. Selon l'exemple 18 de ce brevet US 3.171.831, la gélatine ainsi traitée est utilisée pour encapsuler les produits pharmaceutiques sensibles à l'environnement acide de l'estomac. Le produit pharmaceutique n'est donc pas couplé sur la protéine thiolée mais enrobé par celle-ci.

D'autre part, on a décrit dans la demande de brevet FR 76.37.367 publiée sous le n° 2.334.954 un réactif pour la détermination immunologique enzymatique. Ce réactif est constitué d'un antigène et d'une enzyme couplés par l'intermédiaire d'un ester de l'acide maléimidobenzoïque et du n-hydroxy-succinimide.

On sait que la toxine tétanique est transportée de façon rétrograde vers le système nerveux central et le système nerveux périphérique. A cet effet, on peut se référer à l'article de BIZZINI et al. intitulé : « An antigenic polypeptide fragment isolated from tetanus toxin : chemical characterization, binding to gangliosides and retrograde axonal transport in various neuron systems », paru dans le « journal of Neurochemistry », 1977, vol. 28, pp. 529-542, ainsi qu'à toutes les références bibliographiques citées dans cet article.

Différentes études ont montré que la toxine tétanique pouvait être dégradée ou scindée en plusieurs fractions ou sous-unités. par exemple, COHEN et al. [The Journal of Immunology vol. 104, n° *6* juin 1970] ont montré que la congélation-décongélation de filtrat brut de culture de *Clostridium tetani* entraîne la dégradation de la molécule de la toxine tétanique ; la toxine tétanique dégradée résultante est pratiquement dépourvue de toxicité et présente un pouvoir floculant plus faible que la toxine tétanique.

BIZZINI et RAYNAUD ont également étudié les sous-unités A-I, A-II, A-III, et B-I, B-II et B-III de la toxine tétanique [C.R. Acad. Sc. Paris, t. 279, 1974 série D, pp. 1809-1811 et Annales de l'Institut Pasteur Paris *126*, 159-176 (1975)]. Le brevet FR 74.36.622 (publication 2.249.679) décrit un produit atoxique immunogène obtenu à partir de la toxine tétanique. Ce produit atoxique est obtenu par traitement de la toxine tétanique avec une protéinase.

BIZZINI et al. ont également isolé, à partir de la toxine brute congelée, un fragment polypeptidique de la toxine, qui est identique, du point de vue immunologique, aux fragments A-II et B-II cités ci-dessus,

mais en diffère par sa taille et sa toxicité (voir à cet effet Journal of Neurochemistry, 1977, vol. 28, pp. 529-542). Ce fragment, dénommé B-$II_b$, qui sera défini plus en détail ci-après, est capable de se fixer sur les gangliosides et sur les membranes synaptiques avec une affinité qui est même supérieure à celle de la toxine tétanique.

Compte tenu de cette propriété, il est suggéré dans cet article que ce fragment pourrait être utilisé pour véhiculer spécifiquement dans le système nerveux central des agents chimiothérapeutiques, ou des agents pharmacologiques, pour déterminer des effets spécifiques dans le système nerveux central.

On sait également que le fragment B-$II_b$ peut traverser la synapse. D'autre part, dans des expériences d'infection par le virus rabique il a été montré que le fragment B-$II_b$, s'il est injecté avant le virus, protège l'animal. Il semble donc que le fragment B-$II_b$ interfère avec le virus rabique, soit pour l'attachement sur les sites de fixation pour le virus sur les cellules nerveuses, soit avec le mécanisme de transport du virus.

Cependant on n'a pas jusqu'à présent trouvé le moyen de fixer ces agents chimiothérapeutiques ou pharmacologiques sur ledit fragment B-$II_b$ de manière, d'une part, à maintenir toute l'activité de l'agent à véhiculer vers le système central et, d'autre part, à conserver la propriété de fixation du fragment B-$II_b$ sur les récepteurs pour la toxine tétanique dans le système nerveux central.

On a maintenant trouvé un moyen de fixer des agents chimiothérapeutiques ou pharmacologiques sur le fragment B-$II_b$ tout en lui conservant sa propriété de fixation sur les récepteurs pour la toxine tétanique dans le système nerveux central.

Par ailleurs, il est très difficile, à la connaissance de la demanderesse, de séparer les cellules neurolanes des cellules gliales. On a trouvé que le nouveau composé polypeptidique thiolé selon la présente invention pourrait être utilisé comme marqueur spécifique des cellules neuronales ; la séparation des cellules neuronales marquées devient alors plus aisée par des techniques bien connues de l'homme de l'art, telles que la filtration sur gel, la chromatographie d'affinité, etc...

Le composé polypeptidique thiolé selon la présente invention est constitué par le fragment B-$II_b$ de la toxine tétanique sur lequel est fixé au moins un groupe-SH et présente sensiblement les mêmes propriétés de transport rétrograde axonal et de fixation sur les récepteurs de la toxine tétanique que le fragment B-$II_b$ lui-même.

Dans le composé de l'invention, le ou les groupes-SH sont fixés, directement ou indirectement, sur le fragment B-$II_b$. En général, compte tenu du procédé pour son obtention, qui implique une thiolation, la fixation des groupes-SH se fera par l'intermédiaire du résidu de l'agent de thiolation. Celui-ci est par ailleurs fixé sur le fragment B-$II_b$ par l'intermédiaire des groupes-$NH_2$ dont celui-ci est porteur.

Le fragment B-$II_b$, dont dérive le nouveau composé polypeptidique thiolé selon la présente invention, est un polypeptide de la toxine tétanique dont le poids moléculaire est d'environ 46 000.

Le procédé d'obtention de ce fragment B-$II_b$ ainsi que ses propriétés physico-chimiques et immunologiques sont décrits en détail dans l'article de BIZZINI et al., cité précédemment (Journal of Neurochemistry, 1977, vol. 28, pp. 529-542).

On rappellera brièvement que ce fragment a été obtenu par le procédé qui consiste à soumettre un filtrat congelé de culture de *Clostridium tetani* (souche Harvard n° 6037 de la Collection Nationale de Cultures de Microorganismes de l'Institut Pasteur) aux étapes ci-après :

1. ultrafiltration pour éliminer les substances ayant un poids moléculaire inférieur à 10 000 ;
2. fractionnement à l'aide de sulfate d'ammonium ;
3. filtration sur gel

Le fragment B-$II_b$ présente les propriétés physicochimiques ci-après :

— poids moléculaire d'environ 46 000 ;
— il possède un pont disulfure et deux groupes sulfhydryle libres non réactifs ;
— les groupes N-terminaux sont la tyrosine et la lysine ;
— sa composition en acides aminés est donnée dans le tableau I ci-après.

Le fragment B-$II_b$ de la toxine tétanique possède la même structure antigénique que le fragment B-II mais en diffère par sa toxicité spécifique. Les propriétés immunologiques du fragment B-$II_b$ comparées à celle de la toxine tétanique et du fragment B-II sont rassemblées dans le tableau II ci-après.

(voir tableau I page 4)

TABLEAU I

Composition en acides aminés du fragment $B\text{-}II_b$.

| Acides aminés | Acide aminé (g/100g protéine) | Azote (g/100g protéine) | Nombre de résidus supposés | Poids moléculaire calculé | Acides aminés extracellulaires à la toxine (g/100g protéine) |
|---|---|---|---|---|---|
| Tryptophane | 2,96 | 0,41 | 7 | 48293 | 1,63 |
| Lysine | 7,36 | 1,41 | 23 | 45678 | 10,42 |
| Histidine | 1,61 | 0,44 | 5 | 48185 | 1,34 |
| Amide | -- | 2,21 | -- | -- | -- |
| Arginine | 4,60 | 1,48 | 12 | 45444 | 3,94 |
| Acide aspartique | 21,87 | 2,30 | 76 | 46208 | 17,72 |
| Thréonine | 5,17 | 0,61 | 20 | 46080 | 5,32 |
| Sérine | 7,60 | 1,01 | 33 | 45606 | 6,59 |
| Acide glutamique | 7,83 | 0,74 | 25 | 46975 | 10,49 |
| Pyroline | 3,54 | 0,43 | 14 | 45528 | 4,22 |
| Glycine | 4,14 | 0,77 | 25 | 45325 | 3,30 |
| Alanine | 3,53 | 0,55 | 18 | 45432 | 3,21 |
| Semi-cystine | 0,95 | 0,11 | 4 | 50656 | 1,12 |
| Valine | 6,22 | 0,76 | 24 | 45192 | 4,78 |
| Méthionine | 1,75 | 0,16 | 5 | 42630 | 2,29 |
| Isoleucine | 10,32 | 1,10 | 36 | 45756 | 11,02 |
| Leucine | 11,38 | 1,22 | 40 | 46120 | 10,05 |
| Tyrosine | 10,25 | 0,79 | 26 | 45942 | 9,79 |
| Phénylalanine | 5,80 | 0,49 | 16 | 45568 | 6,39 |
| Total | 116,88 | 16,99 | 409 | 46145 | 113,62 |

TABLEAU II

Comparaison des principales caractéristiques immunologiques et toxiques du fragment B-$II_b$ avec celles du fragment B-II et de la toxine tétanique.

| Composé | Test Ouchterlony (structure antigénique) | Activité floculante spécifique (LF/mgN) | LF/O.D | LF/Lf | Toxicité spécifique LD/mgN |
|---|---|---|---|---|---|
| toxine | 1 (a,b) | 3150 | 423 | $2{,}5.10^3$ | $7{,}8.10^7$ |
| fragment B-$II_b$ | 1 (b) | 11000 | 1000 | 0,05 | 555 |
| fragment B-II | 1 (b) | 8200 | 825 | 8 | $6{,}6.10^4$ |

Le composé polypeptidique thiolé selon la présente invention est obtenu par thiolation du fragment B-$II_b$ traité au préalable pour éliminer sa toxicité résiduelle.

La thiolation du fragment B-$II_b$ peut être effectuée par des moyens classiques permettant l'introduction de groupes-SH sur une molécule comportant des groupes amino, mais, pour les besoins de l'invention, les moyens en cause ne doivent pas dénaturer les propriétés de transport axonal et de fixation sur les récepteurs spécifiques de la toxine tétanique dans le système nerveux central du fragment B-$II_b$.

A titre d'exemple, on indiquera que la thiolation du fragment B-$II_b$ peut être réalisée avec les agents de thiolation ci-après.

4. méthyl-mercaptobutyrimidate :

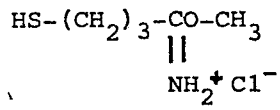

[Biochemistry vol. 17 n° 8, 1978]

2. iminothiolane [Schramm H. J. et Dölffer T. (1977) Z. Physiol. Chem. *358,* 137-139]

N. acétylhomocystéine thiolactone (AHT) [voir J. Am. Chem. Soc., 1960, *82,* 565-571]

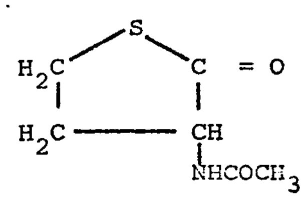

l'anhydride S-acétyl-mercaptosuccinimique (AMS) [J. Am. Chem. Soc. 1959, *81,* 3802-3803]

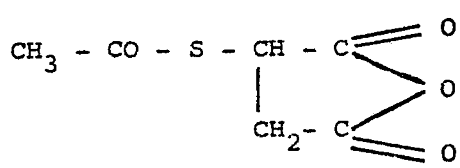

Par contre, on indiquera que les procédés connus de thiolation consistant en une étape de dithiopyridylation et en une étape de réduction ne conviennent pas aux fins de l'invention. En effet, les propriétés de transport axonal et les propriétés de fixation du fragment B-$II_b$ ainsi thiolé sont modifiés au cours de l'étape de réduction.

Par exemple, la thiolation effectuée par réaction avec le N-succinimidyl-3-(2-pyridyl-dithio)-propionate et par réduction du composé dithiopyridylé obtenu, par exemple, selon le mode opératoire décrit par Carlsson et al. [Bioch. J. (1978) *173,* 723-724] n'est pas appropriée aux fins de l'invention.

Pour plus de précision, on indiquera que le composé polypeptidique thiolé selon l'invention comporte un ou plusieurs groupes Z-SH, Z étant le résidu de l'agent de thiolation.

Ainsi, si l'on met en œuvre l'un des agents de thiolation ci-dessus, Z représente alors :

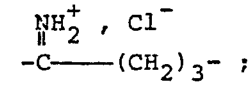 ; 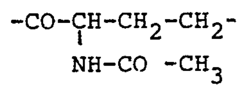 ; 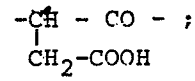 ;

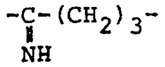

La thiolation du fragment B-$II_b$ est réalisée sur les groupes $NH_2$ de celui-ci.

La toxicité résiduelle du fragment B-$II_b$ peut être éliminée au préalable, par exemple par une étape d'immuno-adsorption sur une colonne de gel de Sépharose 4B marque déposée activé avec du CNBr sur lequel est fixée de façon covalente la fraction IgG du sérum anti-Ibc. Le fragment B-$II_b$ ainsi obtenu n'est pas toxique chez la souris à la dose de 1,9 mg.

On a trouvé que le composé polypeptidique thiolé selon l'invention convient comme agent de transport neuropharmacologique pour véhiculer vers le système nerveux central des agents pharmacologiques ou chimiothérapeutiques.

Pour permettre le transport d'un médicament vers le système nerveux central par l'intermédiaire de l'agent selon l'invention, il est nécessaire de fixer ou de coupler ce médicament sur le composé polypeptidique thiolé, utilisé comme agent de transport, sans évidemment modifier la propriété pharmacologique du médicament ni la propriété de fixation du fragment B-$II_b$ sur les récepteurs spécifiques de la toxine tétanique dans le système nerveux central. Par « médicaments » on désigne, selon l'invention, toutes substances ayant des propriétés pharmacologiques, telles que les agents pharmacologiques, les agents chimiothérapeutiques, etc... Les médicaments qui peuvent être fixés selon l'invention sur le composé polypeptidique utilisé comme agent de transport neuropharmacologique doivent posséder des groupes $NH_2$.

A titre d'exemples de médicaments qui peuvent être véhiculés vers le système nerveux central au moyen du composé polypeptidique thiolé selon l'invention, on citera : la phosphatase alcaline, le fragment A de la toxine cholérique, le fragment A de la toxine diphtérique, les dipyrido-indoles selon le brevet FR 77.11.148 publié sous le n° 2.387.229 et, de façon générale, tout médicament porteur de groupes $NH_2$.

Il est connu que la toxine cholérique se fixe sur les gangliosides $GM_1$ de la paroi intestinale et que le fragment A est responsable de l'augmentation du taux d'AMP cyclique [acide adénosine-monophosphorique cyclique]. Par contre, dans le tétanos, on constate une diminution du taux d'AMP cyclique dans le système nerveux central. Le conjugué selon l'invention, constitué du composé polypeptidique thiolé couplé avec le fragment A, peut être effectué pour lutter contre le tétanos.

Les dipyrido-indoles selon le brevet FR 77.11.148 publié sous le n° 2.387.229 sont des chimiothérapeutiques utiles dans le traitement des cancers. Dans ce domaine, on sait que les métastases sont dues au fait que des cellules cancéreuses viennent se nicher dans le système nerveux central, d'où elles migrent vers d'autres régions du corps, où elles développent des tumeurs. Le développement des métastases pourrait être évité ou réduit dès lors que les moyens pour détruire ces cellules dans le système nerveux central parviendront jusqu'au système nerveux central.

De la même manière, l'invention peut être appliquée au traitement des tumeurs cérébrales.

La présente invention concerne donc également les moyens de couplage du composé polypeptidique thiolé selon l'invention avec des médicaments.

Les moyens de couplage du composé selon l'invention et du médicament à transporter mettent en œuvre au moins un pont disulfure ou au moins une liaison irréversible soufrée.

La présente invention concerne donc également les conjugués fragment B-$II_b$/Médicament comportant au moins un pont disulfure ou au moins une liaison irréversible soufrée.

Il est connu de préparer des conjugués protéiniques par formation de liaison disulfure intermoléculaire. La formation d'une telle liaison disulfure intermoléculaire est réalisée par exemple par réaction d'une protéine ayant des groupes thiol avec une protéine ayant des groupes dithiopyridyle.

Par exemple, selon le procédé décrit par TE PIAO KING et al. (Biochemistry vol. 17 n° 8, 1978) on peut coupler deux protéines différentes en fixant d'abord sur une des protéines des groupes thiol et sur l'autre protéine des groupes 4-dithiopyridyle et en faisant réagir les deux protéines modifiées résultantes dans des conditions appropriées permettant la formation d'un pont disulfure et l'élimination de 4-thiopyridone. Les groupes thiol peuvent être fixés sur l'une des protéines à l'aide de 4-méthyl-mercapto-butyrimidate et les groupes 4-dithiopyridyle sur l'autre protéine par l'intermédiaire de, par exemple, le 3-méthyl-(4'-dithiopyridyl) propionimidate. Ce procédé de couplage permet l'obtention d'un conjugué protéine-protéine dans lequel la fraction entre les deux protéines est symétrique par rapport au pont disulfure.

Selon CARLSSON et al. (Bioch. J ; 1978, *173*, 723-724) on peut introduire le groupe thiol sur l'une des protéines par réaction de ladite protéine avec le N-succinimidyl-3-(2-pyridyl-dithio) propionate et réduction ultérieure ; selon ce procédé, on utilise le même réactif, à savoir le N-succinimidyl-3-(2-pyridyl-dithio) propionate, pour introduire des groupes thio et dithiopyridyle sur les protéines. Les conjugués résultants possèdent également une fraction de liaison symétrique par rapport au pont disulfure.

On a également utilisé le 4-méthyl-mercapto-butyrimidate pour la formation de dimères et d'oligomè-

res supérieurs de protéines du ribosome 30 S d'*Escherichia Coli* (Biochemistry, *12*, 3266-3273, 1973).

Les conjugués ainsi obtenus ont de nombreuses applications, par exemple comme réactifs de dosages immunologiques.

Le procédé de couplage, selon l'invention, du composé polypeptidique thiolé utilisé comme agent de transfert neuropharmacologique avec un médicament par l'intermédiaire de ponts disulfure consiste :

1. à introduire sur le médicament à fixer des groupes dithiopyridyle ;

2. à faire réagir le médicament porteur de groupes dithiopyridyle avec le composé polypeptidique thiolé selon l'invention.

Le schéma réactionnel de ce procédé de couplage peut être représenté de la façon suivante lorsque l'agent de dithiopyridylation mis en œuvre dans l'étape 1 est le N-succinimidyl-3-(2-pyridyl-dithio)propionate :

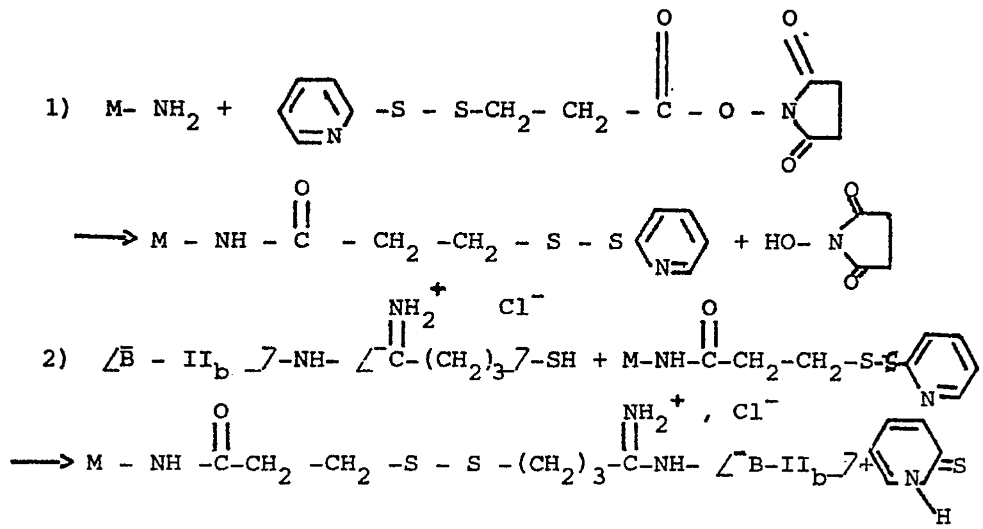

Dans ce procédé on peut utiliser un autre agent de dithiopyridylation, tel que la dithiopyridine ou tout autre agent approprié pour une telle réaction.

Un autre moyen de couplage du composé polypeptidique utilisé comme agent de transport neuropharmacologique selon l'invention consiste à créer une liaison irréversible entre ledit agent et le médicament à véhiculer. Ce procédé peut être représenté par le schéma réactionnel ci-après :

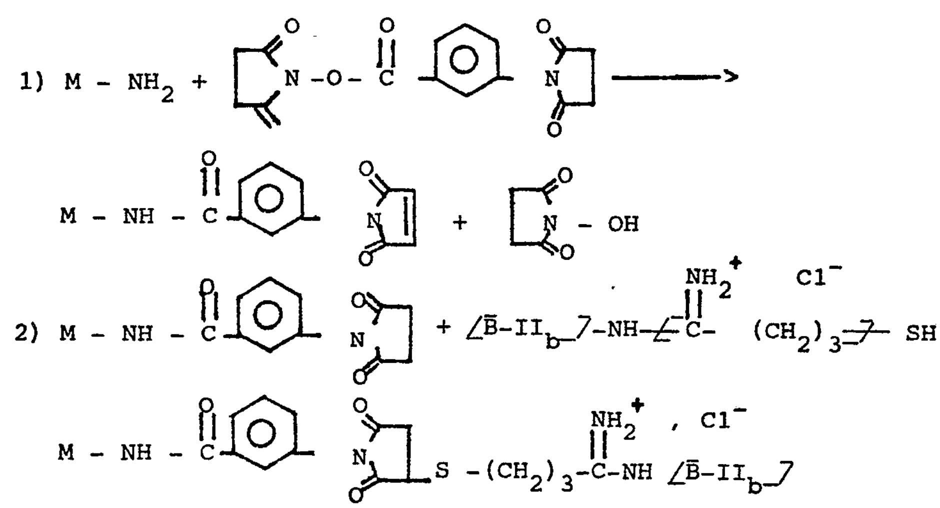

Il consiste :

1. à faire réagir le médicament à fixer avec l'ester de métamaléimidobenzoyl-N-hydroxy-succinimide.

2. à faire régir le composé résultant avec le composé polypeptidique selon l'invention.

Les schémas réactionnels ci-dessus et ceux qui seront donnés ci-après sont simplifiés et ne tiennent pas compte du nombre de groupes SH qui peuvent être fixés sur le fragment $B\text{-}II_b$.

On a déjà proposé d'utiliser l'ester de métamaléimidobenzoyl-N-hydrosuccinimide pour former des conjugués enzyme-anticorps (FEBS Letters, vol. 95, n° 2, Nov. 1978). Toutefois, les enseignements de l'art antérieur ne permettaient pas de prévoir que l'utilisation de l'ester de métamaléimidobenzoyl-N-hydroxysuccinimide pour le couplage de l'agent de transport neuropharmacologique selon l'invention avec des médicaments ne modifierait ou n'inhiberait par les propriétés pharmacologiques dudit

médicament et la propriété de fixation du fragment B-$II_b$ sur les récepteurs spécifiques de la toxine tétanique dans le système nerveux central.

On remarquera que le couplage selon l'invention du médicament avec le composé polypeptidique thiolé utilisé comme agent de transport neuropharmacologique est réalisé selon des techniques connues classiques de couplage protéine-protéine. Toutefois, il faut noter que toutes les techniques de couplage protéine-protéine à la portée de l'homme de l'art ne conviennent pas aux fins de l'invention. En effet, seules les techniques de couplage permettant de réaliser une liaison disulfure ou une liaison irréversible soufrée sont appropriées. En particulier, on indiquera que la technique de couplage la plus classique, qui met en œuvre le glutaraldéhyde, ne convient pas aux fins de l'invention car le fragment B-$II_b$ traité par le glutaraldéhyde perd ses propriétés de transport axonal et de fixation sur les récepteurs de la toxine tétanique dans le système nerveux central. Ainsi, le fragment B-$II_b$, sur lequel on aurait fixé des groupes carbonyle, avec le glutaraldéhyde par exemple, ne convient pas aux fins de l'invention.

Une autre application du composé polypeptidique thiolé selon l'invention est le marquage des cellules neuronales. Ainsi, le composé de l'invention peut être utilisé comme marqueur spécifique des cellules neuronales et aussi pour préparer des réactifs immunologiques. On peut préparer par exemple un réactif enzymatique à partir du composé polypeptidique thiolé selon l'invention et de la phosphatase alcaline. On a constaté que le conjugué résultant possédait à la fois le pouvoir de fixation du fragment B-$II_b$ et l'activité enzymatique de la phosphatase. Le composé selon l'invention convient aussi comme traceur rétrograde transsynaptique.

L'invention va être maintenant décrite plus en détail par les exemples illustratifs de préparation du composé polypeptidique thiolé de l'invention et de couplage de celui-ci, avec des médicaments. Dans tous ces exemples on a utilisé le fragment B-$II_b$ tel que défini ci-dessus, dépourvu de sa toxicité résiduelle.

## Exemple 1

Thiolation du fragment B-$II_b$ à l'aide du 2-iminothiolane

La thiolation du fragment B-$II_b$ a été réalisée selon le mode opératoire décrit par SCHRAMM et al. [Z. Physiol. Chem. 1977, *358*, 137-139].

Le fragment B-$II_b$ (1 mg) en solution dans du glycérol à 50 % (0,2 ml) a été thiolé par l'iminothiolane (0,5 mg) en solution dans 750 μl de tampon triéthanolamine HCl 0,2 M, pH 8,5-9,0. Le mélange réactionnel a été maintenu à la température ambiante pendant deux heures. L'excès de réactif a été éliminé par filtration sur une colonne « SEPHADEX G-25 ». Le produit polypeptidique thiolé ainsi obtenu contenait 1,2 groupe —SH.

## Exemple 2

Thiolation du fragment B-$II_b$ à l'aide de la N-acétylhomocystéine thiolactone.

On a réalisé la thiolation du fragment B-$II_b$ selon le mode opératoire décrit par SINGER et al. [J. Am. Chem. Soc. 1960, *82*, 567-571].

A 3 mg du fragment B-$II_b$ en solution dans 2 ml d'eau on a ajouté le tampon $K_2CO_3/NaHCO_3$ pH 10,7 (1,6 ml). On a fait passer un courant d'azote pour chasser l'air. On a ajouté 0,4 ml d'une solution de N-acétylhomocystéine-thiolactone à 80 mg/l. On a maintenu le mélange réactionnel pendant 2 heures à 4 °C sous azote. La réaction a été ensuite arrêtée par filtration sur SEPHADEX G-25. Le produit obtenu contenait 2,8 groupes —SH.

## Exemple 3

Thiolation du fragment B-$II_b$ à l'aide de l'anhydride S-acétyl-mercaptosuccinimique

On a réalisé la thiolation du fragment B-$II_b$ selon le mode opératoire décrit par KLOTZ et al. [J. Am. Chem. Soc. 1959, *81*, 3802-3803].

On a introduit le fragment B-$II_b$ (3 mg) dans 1 ml de tampon phosphaté 0,1 M, pH 7,4 dans un ballon à trois tubulures et on a purgé le ballon avec de l'azote.

Sous agitation, et en maintenant une atmosphère d'azote, on a introduit 140 μg de l'anhydride S-acétyl-mercaptosuccinimique en deux fois à 5 minutes d'intervalle.

On a laissé la réaction s'accomplir pendant 20 minutes au total. On a ensuite filtré le mélange réactionnel sur SEPHADEX G-25. On a recueilli une solution du fragment B-$II_b$ modifié à laquelle on a ajouté du $NH_2OH$ 1 M jusqu'à l'obtention d'une concentration finale de 0,1 M. On a laissé la désacétylation s'effectuer pendant 5 minutes. On a ensuite filtré immédiatement le mélange réactionnel sur du SEPHADEX G-25 équilibré en tampon phosphate 0,1 M pH 7,4.

Le schéma réactionnel peut être représenté comme suit :

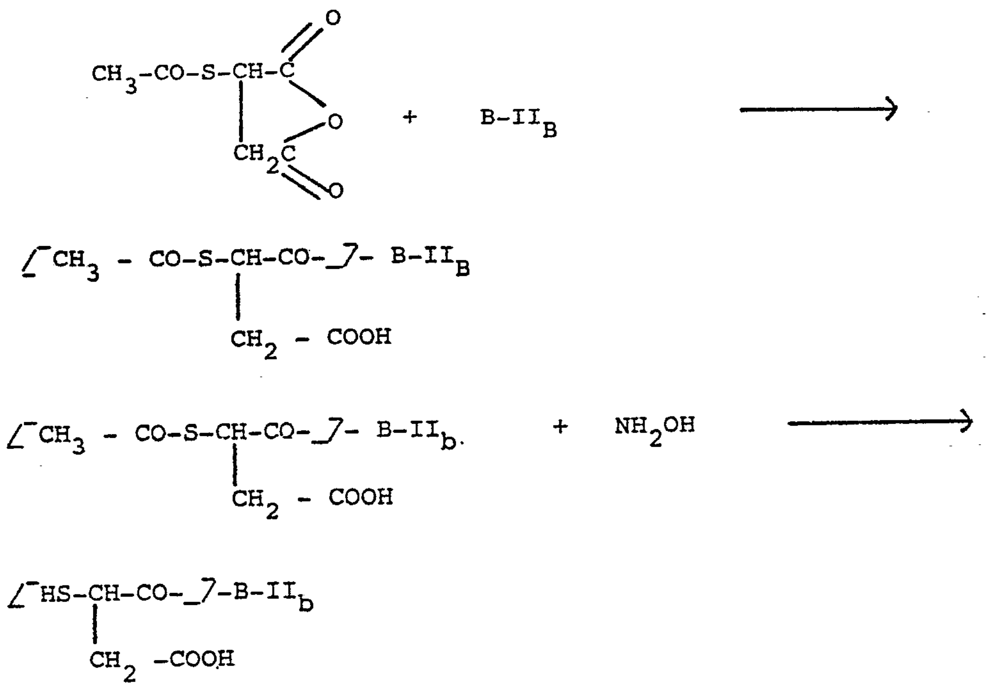


Le produit obtenu contenait 5,6 groupes thiol.

## Exemple 4

Thiolation du fragment B-$II_b$ à l'aide du 4-méthyl-mercaptobutyrimidate.

On a effectué la thiolation selon le mode opératoire décrit par TE PIAO KING et al. [Biochemistry vol. 17 n° 8 1978] à l'aide de 4-méthyl-mercaptobutyrimidate et on a obtenu un composé polypeptidique thiolé comportant 1,4 groupe

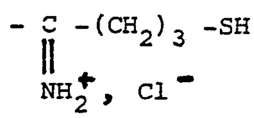

## Exemple 5

Préparation d'un conjugué composé polypeptidique thiolé-fragment A de la toxine diphtérique

On a utilisé 2,2 mg du fragment A de la toxine diphtérique dans 1 ml de tampon borate 0,025 M pH 9,0. On a ajouté 7 mg de 2-iminothiolane et 77 µg de 4,4'-dithiopyridine sous forme d'une solution méthanolique (100 µl d'une solution de 3,85 mg dans 5 ml de méthanol). On a effectué l'addition de cette solution en deux fois 50 µl, à trois minutes d'intervalle, en refroidissant dans la glace et sous agitation. On a laissé la réaction s'accomplir pendant deux heures. On a appliqué aussitôt le mélange réactionnel sur du SEPHADEX G-25 (marque déposée) (0,9 × 20 cm) équilibré avec un tampon phosphate 0,1 M pH 6,9 contenant 1 mM $Na_2$ EDTA.

On a mélangé 3 moles du composé polypeptidique obtenu selon l'exemple 4 avec 2,8 moles du fragment A-dithiopyridylé selon le mode opératoire ci-dessus. On a suivi la réaction d'échange à 324 nm. On a filtré sur SEPHAROSE 6 B (marque déposée) équilibré avec un tampon tris 0,05 M, NaCl 0,5 M, pH 8,0.

Le conjugué obtenu conserve le pouvoir de fixation du fragment B-$II_b$ et l'activité immunologique du fragment A de la toxine diphtérique. On peut ainsi préparer une chimère.

La dithiopyridylation du fragment A peut également être effectuée à l'aide du N-succinimidyl-3-(2-pyridyldithio) propionate.

## Exemple 6

Utilisation du composé polypeptidique thiolé selon l'invention comme agent de transport neuropharmacologique.

Pour montrer que le composé polypeptidique thiolé selon l'invention peut être utilisé comme agent neuropharmacologique spécifique, on a utilisé comme modèle expérimental le système oculomoteur périphérique chez le rat.

On a utilisé le composé polypeptidique thiolé obtenu selon l'exemple 4 ci-dessus. Ce composé polypeptidique thiolé a été couplé avec le fragment $I_{bc}$ de la toxine tétanique.

Le fragment $I_{bc}$ a été obtenu par digestion papaïnique de la toxine.

L'injection à des souris de ce fragment $I_{bc}$ provoque des symptômes d'empoisonnement semblables à ceux du fragment B [HELTING et al. J. Biol. Chem., 253 (1978) 125-129].

On a utilisé le fragment $I_{bc}$ couplé avec le composé polypeptidique selon l'invention pour bien mettre en évidence que c'est le fragment B-$II_b$ qui se fixe sur les récepteurs spécifiques de la toxine tétanique dans le système nerveux central et non pas un autre fragment de la toxine tétanique, tel que le fragment $I_{bc}$. En effet, le conjugué B-$II_b$/$I_{bc}$ comportant des ponts disulfure obtenu selon le procédé de couplage défini ci-dessus ressemble, du point de vue de la structure, à la toxine tétanique entière, et les résultats relatés ci-après indiquent que c'est seulement le fragment B-$II_b$ qui se fixe sur les récepteurs spécifiques de la toxine tétanique.

Les fragments B-$II_b$ et $I_{bc}$ ont été marqués à l'iode radioactif $I^{125}$ ainsi que le conjugué B-$II_b$/$I_{bc}$. Ce marquage a été réalisé selon la méthode de GREENWOOD et al. [Biochem. J. 89, (1963) 114-127].

Pour chaque substance à marquer on a utilisé 400 µg de ladite substance et 2mVi de Na $I^{125}$ (fourni par le Centre de Radiochimie de Amersham). La radioactivité spécifique de chaque substance ainsi marquée est indiquée ci-après :

| Substance | Radioactivité |
|---|---|
| — fragment B-$II_b$ | 3,4 µCi/µg |
| — fragment $I_{bc}$ | 2,6 µCi/µg |
| — conjugué B-$II_b$/$I_{bc}$ | 3 µCi/µg |

Protocole d'essai

Pour tous les essais, on a utilisé des rats Sprague-Dawley femelles d'un poids de 250-270 g. Les rats ont été maintenus à la température constante de 23 °C et nourris avec le régime habituel [Nafag, Gossau] et de l'eau.

Cinq rats albinos ont reçu une injection de l'une des substances marquées ci-dessus dans le muscle médian droit de l'œil gauche selon le mode opératoire ci-après. Après mise à nu du muscle, les substances ont été injectées par un système d'injection réglé thermiquement à l'aide d'une pipette en verre (50-100 µm de diamètre extérieur). Le temps d'injection a été de 35 à 50 minutes. Un rat a reçu le conjugué ci-dessus et quatre autres rats ont reçu l'un des fragments $I_{bc}$ ou B-$II_b$. De la peroxydase de raifort 30 % (HRP type Sigma VI) a été utilisée à titre de témoin. Les rats ont été sacrifiés 24 heures après l'injection. Une perfusion intracardiale a été effectuée sous anesthésie générale avec tout d'abord un expanseur du plasma (« MACRODEX ») (marque déposée), puis avec du glutaraldéhyde à 1,25 % et du paraformaldéhyde à 1 % dans un tampon phosphate 0,1 M (pH 7,4) pendant 30 minutes, et finalement avec une solution de saccharose à 10 % dans un tampon phosphate 0,1 M (pH 7,4) pendant 30 minutes. Pour plus de détail sur ce mode opératoire, on pourra se reporter à l'article de MESULAM [J. Histochem. Cytochem., *26* (1978) 106-117].

Le cerveau a été prélevé immédiatement après cette perfusion et maintenu pendant 44 heures dans une solution de saccharose à 30 % avant découpage. Des sections congelées (épaisseur 30 µm) ont été prélevées à partir du noyau abducteur et jusqu'au noyau oculomoteur entier. Chaque section traitée avec la peroxydase de raifort a été colorée selon la méthode TMB selon MESULAM et recolorée avec du rouge neutre, alors que les autres sections ont été autoradiographiées. Ces dernières sections ont été montées et trempées dans une émulsion liquide $NTB_2$ à 45 °C dilué au 1/2 avec de l'eau distillée. Les sections ont été exposées pendant 4 semaines à 4 °C dans le noir et développées avec le révélateur « Kodak Dektol » (marque déposée) à 18 °C pendant 90 secondes, lavées, puis fixées avec du thiosulfate de sodium à 30 %, lavées pendant 2 heures, puis colorées avec du violet de crésyle et couvertes. Toutes les sections ont été examinées au microscope (grossissement 250) et la localisation des cellules marquées a été déterminée par microphotographie.

Dans une première série d'essais, on a injecté simultanément le conjugué B-$II_b$/$I_{bc}$ avec la peroxydase de raifort dans le muscle médian droit et on a constaté un marquage radioactif dans le noyau oculomoteur pour le conjugué et le peroxydase de raifort.

La principale différence entre la peroxydase de raifort (HRP) et le conjugué B-$II_b$/$I_{bc}$ consiste en le fait que la localisation des granules de HRP est limitée aux péricaryons et dendrites du noyau oculomoteur, tandis que les grains d'argent qui représentent le conjugué marqué avec $I^{125}$ ont été trouvés dans les espaces péricellulaires.

Dans une autre série d'essais, on a injecté à deux rats, selon le mode opératoire ci-dessus, le fragment B-$II_b$ marqué par $I^{125}$ et on a observé des cellules marquées dans les neurones du noyau

oculomoteur ipsilatéral chez les deux animaux ainsi traités.

Dans une troisième série d'essais, on a injecté simultanément le fragment $I_{bc}$ marqué avec $I^{125}$ et HRP dans le muscle de l'œil d'un rat. Cette fois-ci, on a observé uniquement un marquage des cellules du noyau oculomoteur dû à la peroxydase de raifort. Chez un autre animal, on a injecté seulement le fragment $I_{bc}$ marqué par $I^{125}$ et on n'a observé aucun marquage des cellules du noyau oculomoteur. Le fait que le test réalisé avec le premier animal (traité simultanément avec HRP et $I_{bc}$ marqué par $I^{125}$) ait donné des résultats positifs dus à HRP est important, car il montre que le fait que le fragment $I_{bc}$ ne soit pas transporté de façon axonale rétrograde ne résulte pas d'une rupture du flux axonal rétrograde, mais plutôt des propriétés spécifiques du fragment $I_{bc}$ qui semblent être incompatibles avec les fonctions de transport intraaxonal.

Par ailleurs, on a trouvé que le fragment $I_{bc}$ ne se lie pas aux gangliosides et membranes synaptiques isolées. Par contre, le fragment B-$II_b$ se lie de façon spécifique sur les gangliosides et les membranes synaptiques [voir Journal of Neurochemistry (1977), vol. *28*, p. 529-542].

Les essais ci-dessus montrent que, pour qu'il y ait transport rétrograde axonal d'une substance, il faut que la substance puisse se lier spécifiquement aux gangliosides et membranes synaptiques.

On a alors vérifié, en utilisant les tests décrits dans Journal of Neurochemistry (1977) vol. *28* p. 529-542, que les conjugués B-$II_b$/Médicament obtenus selon l'invention à partir du composé polypeptidique thiolé se fixaient de façon spécifique sur les gangliosides et les membranes synaptiques ; ces résultats prouvent que ces substances peuvent donc être transportées de façon rétrograde axonale.

La possibilité d'utiliser le fragment B-$II_b$ thiolé de la toxine tétanique comme traceur rétrograde transsynaptique a été exploré chez le singe. Le fragment B-$II_b$ marqué à l'iode 125 a été injecté dans le muscle médian droit de l'œil dont on connaît bien les connexions de ses neurones moteurs et en particulier les insertions à partir des neurones moteurs oculaires externes internucléaires et des noyaux vestibulaires. Les essais effectués montrent que le fragment B-$II_b$ thiolé est bien transporté à travers la synapse puisqu'on peut le détecter dans les corps cellulaires à la fois des neurones primaires et secondaires.

(voir tableau III page 12)

TABLEAU III

Résumé des essais de transport axonal rétrograde

| Essais | Référence de l'essai | Marqueur Radioactif | Concentration (µg/µl) | Volume de l'injection (µl) | Radioactivité spécifique (µCi/ µg) | Animal sacrifié après | Transport rétrograde |
|---|---|---|---|---|---|---|---|
| I | 79-477 | Conjugué B-II$_b$/I$_{bc}$ | 3 | 1,44 | 3 | 24 h | + |
| | . | HRP | 300 | 0,96 | - | - | + |
| II | 79-196 | Fragment $^{125}$I-B-II$_b$ | 3 | 1,00 | 3,4 | 24 h | + |
| | 79-197 | Fragment $^{125}$I-B-II$_b$ | 3 | 1,00 | 3,4 | 24 h | + |
| III | 79-480 | Fragment $^{125}$I-I$_{bc}$ | 3 | 1,44 | 2,6 | 24 h | - |
| | | HRP | 300 | 0,96 | - | - | + |
| | 79-479 | Fragment $^{125}$I-I$_{bc}$ | 3 | 2,40 | 2,6 | 24 h | - |

**Revendications**

1. Composé polypeptidique thiolé constitué par le fragment B-$II_b$ de la toxine tétanique, sur lequel est fixé, directement ou indirectement, au moins un groupe —SH réactif.

2. Composé polypeptidique thiolé selon la revendication I, caractérisé en ce qu'il est obtenu par thiolation et en ce qu'il comporte un ou plusieurs groupes Z-SH, Z étant le résidu de l'agent de thiolation.

3. Composé selon l'une des revendications 1 ou 2, caractérisé en ce que Z est choisi parmi les groupes ci-après :

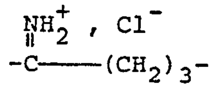 ; 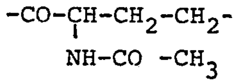 ; 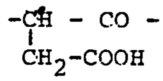 ;

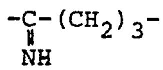

4. Procédé pour l'obtention du composé selon l'une quelconque des revendications 1 à 3, caractérisé en ce qu'on effectue la thiolation du fragment B-$II_b$ à l'aide d'un agent de thiolation dans des conditions telles que les propriétés de transport rétrograde axonal et de fixation sur les récepteurs spécifiques de la toxine tétanique dans le système nerveux central du fragment B-$II_b$ ne soient pas dénaturées.

5. Procédé selon la revendication 4, caractérisé en ce que l'agent de thiolation est choisi parmi :

le 4-méthyl-mercaptobutyrimidate,
le 2-iminothiolane,
la N-acétylhomocystéine thiolactone,
l'anhydride S-acétyl-mercaptosuccinimique.

6. Application du composé polypeptidique thiolé selon l'une quelconque des revendications 1 à 3 à titre d'agent de transport neuropharmacologique spécifique pour véhiculer un médicament dans le système nerveux central.

7. Application du composé polypeptidique thiolé selon l'une quelconque des revendications 1 à 3 à titre de marqueur spécifique des cellules neuronales.

8. Application du composé polypeptidique thiolé selon l'une quelconque des revendications 1 à 3, à titre de traceur rétrograde transsynaptique.

**Claims**

1. Thiolated polypeptide compound constituted by the B-$II_b$ fragment of tetanus toxin to which at least one reactive -SH group is directly or indirectly bound.

2. Thiolated polypeptide compound according to claim 1, characterized in that it is obtained by thiolation and it comprises one or more Z-SH groups, wherein Z is the residue of the thiolation agent.

3. Compound according to anyone of claims 1 or 2, wherein Z is selected from the following groups :

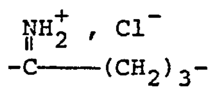 ; 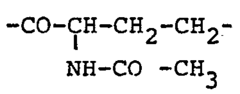 ; 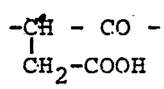 ;

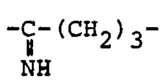

4. Process for obtaining the compound according to anyone of claims 1 to 3, wherein the thiolation of the B-$II_b$ fragment is effected by means of a thiolation agent under such conditions that the properties of retrograde axonal transport and binding to the specific receptors of the tetanus toxin in the central nervous system, of the B-$II_b$ fragment is not denatured.

5. Process according to claim 4, wherein the thiolation agent is selected from the group constituted by :

4-methyl-mercaptobutyrimidate,
2-iminothiolane,
N-acetylhomocysteine thiolactone,
S-acetyl-mercaptosuccinimic acid anhydride.

6. Application of the thiolated polypeptide compound according to anyone of claims 1 to 3, as a specific neuropharmacological transport agent for transporting a medicine to the central nervous system.

7. Application of the thiolated polypeptide compound according to anyone of claims 1 to 3, as a specific labeller of neuronal cells.

8. Application of the thiolated polypeptide compound according to anyone of claims 1 to 3, as retrograde transsynaptic tracer agent.

**Ansprüche**

1. Thiolierte Polypeptidverbindung, bestehend aus dem Fragment B-$II_b$ des Tetanustoxins, an das direkt oder indirekt wenigstens eine reaktionsfähige —SH-Gruppe gebunden ist.

2. Thiolierte Polypeptidverbindung nach Anspruch 1, dadurch gekennzeichnet, daß sie durch Thiolierung erhalten worden ist und eine oder mehrere Gruppen Z-SH enthält, worin Z der Rest des Thiolierungsmittels ist.

3. Verbindung nach einem der Ansprüche 1 oder 2, dadurch gekennzeichnet, daß Z aus den nachstehend genannten Gruppen ausgewählt ist :

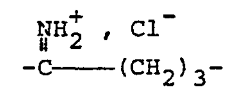 ; 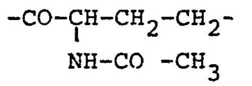 ; 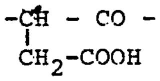 ;

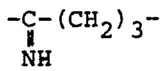

4. Verfahren zur Herstellung der Verbindung gemäß irgendeinem der Ansprüche 1 bis 3, dadurch gekennzeichnet, daß man die Thiolierung der Fragments B-$II_b$ mit Hilfe eines Thiolierungsmittels unter solchen Bedingungen durchführt, daß die Eigenschaften des axonalen rückläufigen Transports und der Bindung an die spezifischen Rezeptoren des Tetanustoxins im Zentralnervensystem des Fragments B-$II_b$ nicht denaturiert werden.

5. Verfahren nach Anspruch 4, dadurch gekennzeichnet, daß das Thiolierungsmittel aus

4-Methylmercaptobutyrimidat,
2-Iminothiolan,
N-Acetylhomocystein thiolacton und
dem S-Acetyl-mercaptobernsteinsäureanhydrid

ausgewählt ist.

6. Anwendung der thiolierten Polypeptidverbindung nach irgendeinem der Ansprüche 1 bis 3 als spezifisches neuropharmakologisches Transportmittel für die Übertragung eines Medikaments im Zentralnervensystem.

7. Anwendung der thiolierten Polypeptidverbindung nach irgendeinem der Ansprüche 1 bis 3 als spezifisches Markierungsmittel der neuronalen Zellen.

8. Anwendung der thiolierten Polypeptidverbindung nach irgendeinem der Ansprüche 1 bis 3 als transsynaptischer retrograder Tracer.